(19) Europäisches Patentamt
European Patent Office
Office européen des brevets

(11) **EP 1 066 335 B1**

(12) **FASCICULE DE BREVET EUROPEEN**

(45) Date de publication et mention de la délivrance du brevet:
**02.07.2003 Bulletin 2003/27**

(21) Numéro de dépôt: **99911860.7**

(22) Date de dépôt: **01.04.1999**

(51) Int Cl.[7]: **C08G 75/04**

(86) Numéro de dépôt international:
**PCT/FR99/00766**

(87) Numéro de publication internationale:
**WO 99/051663 (14.10.1999 Gazette 1999/41)**

(54) **PRODUITS SOUFRES D'ADDITION D'UN POLYTHIOL SUR UN DERIVE DE NORBORNENE, PROCEDE DE FABRICATION ET APPLICATION A L'OBTENTION DE PRODUITS DE RETICULATION PAR VOIE RADICALAIRE**

SCHWEFELENTHALTENDE ADDITIONSVERBINDUNGEN VON POLYTHIOLEN UND NORBORNENDERIVATEN, VERFAHREN ZU IHRER HERSTELLUNG UND IHRER ANWENDUNG ZUR GEWINNUNG VON MITTELS RADIKALBILDUNG VERNETZEN PRODUKTEN

SULPHUR PRODUCTS FOR ADDING A POLYTHIOL ON A NORBORNENE DERIVATIVE, PREPARATION METHOD AND USE FOR OBTAINING RADICAL CROSS-LINKING PRODUCTS

(84) Etats contractants désignés:
**AT BE CH DE DK ES FI FR GB IE IT LI NL PT SE**

(30) Priorité: **03.04.1998 FR 9804166**

(43) Date de publication de la demande:
**10.01.2001 Bulletin 2001/02**

(73) Titulaire: **CRAY VALLEY SA**
**92800 Puteaux (FR)**

(72) Inventeurs:
- **THEPOT, Philippe**
  **F-60140 Liancourt (FR)**
- **STRUB, Henri**
  **F-60700 Pont Sainte Maxence (FR)**

(74) Mandataire: **Chaillot, Geneviève**
**Cabinet CHAILLOT,**
**16-20, avenue de L'Agent Sarre,**
**B.P. 74**
**92703 Colombes Cédex (FR)**

(56) Documents cités:
**US-A- 3 734 968**

- **DATABASE CHEMABS CHEMICAL ABSTRACTS SERVICE, COLUMBUS, OHIO, US STN, accession no. 114:248656, XP002090932 -& JP 02 294322 A (SHOWA HIGHPOLYMER CO LTD)**

Remarques:
Le dossier contient des informations techniques présentées postérieurement au dépôt de la demande et ne figurant pas dans le présent fascicule.

EP 1 066 335 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

## Description

La présente invention porte sur de nouveaux produits soufrés d'addition d'au moins un polythiol sur au moins un dérivé de norbornène comportant une insaturation éthylénique exocyclique ; sur un procédé de fabrication de ces produits soufrés ; sur une composition réticulable par voie radicalaire contenant un tel produit soufré à l'état réticulable, ainsi que sur les produits réticulés résultants et leur fabrication.

L'invention vise à élargir la gamme de ces produits soufrés qui présentent de l'intérêt dans divers domaines : matériaux optiques, revêtements et adhésifs, et dont on peut faire varier les propriétés mécaniques en fonction du polythiol.

En particulier, on souhaite obtenir, pour des applications optiques, des produits qui soient réticulables par voie radicalaire, notamment par voie photochimique, pour donner des matériaux réticulés à indice de réfraction élevé, en visant plus spécialement dans ce cas des produits ayant la teneur la plus élevée possible en soufre pour améliorer encore l'indice de réfraction de ces matériaux.

Ont été décrits d'une manière générale des produits de réaction de polythiols et de composés alicycliques insaturés ayant au moins deux insaturations par molécule, au moins l'une d'elles étant une insaturation cyclique.

Cependant, des expériences conduites par la Société déposante ont permis de constater que :

- la réticulation directe d'un mélange de polythiol et de vinylnorbornène n'est pas toujours possible: le mélange de tri- ou tétrathiols testés avec le vinyinorbornène était un mélange biphasique ; les dithiols testés ont donné un mélange homogène avec le vinylnorbornène, mais l'adjonction d'un photoinitiateur ne permet pas la réticulation sous UV ;
- la réaction par voie thermique d'un polythiol et du dicyclopentadiène avec un groupement thiol par mole de dicyclopentadiène conduit à un produit qui ne réticule pas sous UV avec un polythiol comme agent réticulant.

C'est dans ces circonstances que la Société déposante a découvert que, de façon surprenante, une famille particulière de composés, à savoir des norbornènes substitués comportant une insaturation exocyclique, donne avec les polythiols, dans des conditions de stoechiométrie d'un ou d'environ un groupe thiol pour une mole du norbornène en question, des produits d'addition réticulables par la suite sous UV, et, d'une manière générale, par voie radicalaire, en présence d'au moins un polythiol.

On peut ainsi préparer des matériaux fortement soufrés, répondant aux objectifs visés, en préparant d'abord un produit soufré (P) réticulable, liquide, qui constituera la matière première pour la formulation d'un produit (P') ou d'une composition réticulable sous la forme voulue : film ou pièce moulée. Ledit produit soufré (P) ne présente pas le désavantage des polythiols et des dérivés de norbornène à partir desquels il est obtenu et qui sont des produits volatils, ayant une odeur désagréable et présentant une toxicité. Le formulateur du produit (P') ou de la composition réticulable manipulera alors des produits sans odeur ou à très faible odeur et non toxiques.

On connaît, par DATABASE CHEMABS CHEMICAL ABSTRACTS SERVICE, COLUMBUS, OHIO, US, STN, accession no. 114:248656, XP002090932, l'utilisation de produits d'addition d'éthylidène-5 norbornène-2 et de tétrakis (mercaptoacétate-3)pentaérythritol avec un rapport molaire 0,5/1 (soit 4 fonctions -SH par double liaison), comme agent de réticulation multifonctionnel (polythiol).

Par le brevet américain US-A-3 734 968, on connaît des produits de réaction (dithiols) du vinylcyclohexène sur un dithiol.

La préparation de produits d'addition stables polythiol/diène type norbornène portant des doubles liaisons terminales et réticulables soit par addition supplémentaire de polythiol soit par polymérisation radicalaire, n'est donc pas envisagée par ces deux documents antérieurs.

La présente invention a donc d'abord pour objet un produit soufré obtenu par la réaction d'addition d'au moins un polythiol (I)

(A) sur au moins un composé de formule (II) :

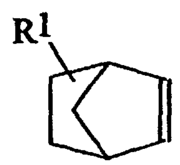

(II)

dans laquelle $R^1$ représente un reste aliphatique, linéaire ou ramifié, comportant une double liaison d'insaturation

éthylénique, cette dernière pouvant être celle par laquelle $R^1$ est relié au cycle norbornène, ledit cycle pouvant également comporter d'autres substituants choisis parmi les groupements alkyle en $C_1$-$C_3$, dans des proportions correspondant ou correspondant sensiblement à un groupement thiol du ou des polythiols (I) par mole du ou des composés (II), les groupes thiol du ou des composés (I) s'étant additionnés majoritairement sur la double liaison cyclique du ou des composés (II) et, en moyenne, une insaturation éthylénique d'un composé (II) sur les deux qu'il porte étant restée libre ; ou

(B) sur au moins un produit (P) réticulable, tel qu'obtenu conformément au point (A) ci-dessus, les doubles liaisons dudit produit (P) ayant été totalement ou sensiblement totalement consommées, le produit (P') obtenu étant un produit réticulé.

Le ou les polythiols (I) ayant été utilisés pour former le produit réticulé (P') ne sont pas nécessairement les mêmes que ceux qui ont servi à former le produit réticulable (P).

Le produit réticulé (P') est obtenu avec un polythiol (I) de fonctionnalité SH d'au moins 2 dans le cas où le produit réticulable (P) a une fonctionnalité en insaturations éthyléniques d'au moins 3 et avec un polythiol (I) de fonctionnalité SH d'au moins 3 dans le cas où le produit réticulable (P) a une fonctionnalité en insaturations éthyléniques d'au moins 2. Dans le cas où l'on utilise des mélanges de polythiols pour la réticulation, leur fonctionnalité moyenne SH sera d'au moins 2.

En particulier, le composé de formule (II) est le 5-vinyl-2-norbornène (5-vinylbicyclo[2.2.1]hepto-2-ène) ou le 5-éthylidène-2-norbornène (5-éthylidène-bicyclo[2.2.1]hepto-2-ène).

Les polythiols (I) sont notamment choisis parmi les composés de formule $R^2$-[-SH]$_n$ dans laquelle :

- $R^2$ est un radical n-valent aliphatique, aromatique ou hétérocyclique ou comportant une combinaison de ceux-ci, $R^2$ pouvant comporter des substituants, le squelette ou les substituants pouvant être interrompus par au moins un hétéroatome choisi parmi -O- ou -S- et/ou un groupement choisi parmi

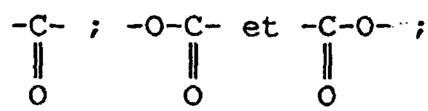

et

- n est un entier de 2 à 6.

Les polythiols (I) peuvent être des polythiols obtenus par addition d'un polythiol de formule $R^2$-[-SH]$_n$, dans laquelle $R^2$ et n sont tels que définis ci-dessus, sur un composé de formule (II) telle que définie ci-dessus, avec un excès de thiol et dans des conditions d'addition évitant une réticulation, sur la base de la relation de Makosco-Miller (Marcomolecules, 1976, volume 9, pages 199-211).

Des exemples de polythiols comprennent :

(1) les polythiols aliphatiques, tels que le méthanedithiol, le 1,2-éthanedithiol, le 1,1-propanedithiol, le 1,3-propanedithiol, le 2,2-propanedithiol, le 1,6-hexanedithiol, le 1,2,3-propanetrithiol, le 1,1-cyclohexanedithiol, le 1,2-cyclohexanedithiol, le 2,2-diméthylpropane-1,3-dithiol, le 3,4-diméthoxybutène-1,2-dithiol, le bicyclo[2.2.1]-hepta-exo-cis-2,3-dithiol, le 1,1-bis(mercaptométhyl)-cyclohexane, l'acide thiomalique bis(2-mercaptoéthyl ester), l'acide 2,3-dimercaptosuccinique (2-mercaptoéthyl ester), le 2,3-dimercapto-1-propanol (2-mercaptoacétate), le 2,3-dimexcapto-1-propanol (3-mercaptopropionate), le diéthylèneglycol bis(2-mercaptoacétate), le diéthylèneglycol bis (3-mercaptopropionate), le 1,2-dimercaptopropyl méthyl éther, le 2,3-dimercaptopropyl méthyl éther, le 2,2-bis (mercaptométhyl)-1,3-propanedithiol, le bis(2-mercaptoéthyl)éther, l'éthylène glycol bis(2-mercaptoacétate), l'éthylène glycol bis(3-mercaptopropionate), le triméthylolpropane tris(3-mercaptopropionate), le triméthylolpropane tris(2-mercaptoacétate), le pentaérythritol tétrakis(3-mercaptopropionate), le pentaérythritol tétrakis(2-mercaptoacétate), les polyéthylène glycol dimercaptoacétates et les polyéthylène glycol di(mercaptopropionates), et ces mêmes composés substitués par halogène, tel que le chlore et le brome;

(2) les polythiols aromatiques, tels que le 1,2-dimercaptobenzène, le 4-t-butyl-1,2-benzènedithiol, le 1,3-dimercaptobenzène, le 1,4-dimercaptobenzène, le 1,2-bis-(mercaptométhyl)benzène, le 1,3-bis(mercaptométhyl)benzène, le 1,4-bis(mercaptométhyl)benzène, le 1,2-bis(mercaptoéthyl)benzène, le 1,3-bis(mercaptoéthyl)benzène, le 1,4-bis(mercaptoéthyl)benzène, le 1,2-bis(mercaptométhylèneoxy)benzène, le 1,3-bis(mercaptométhylèneoxy)benzène, le 1,4-bis(mercaptométhylèneoxy)benzène, le 1,2-bis(mercaptoéthylènoxy)-benzène, le 1,3-bis(mercaptoéthylèneoxy)benzène, le 1,4-bis(merçaptoéthylèneoxy)benzène, le 1,2,3-trimercaptobenzène, le 1,2,4-trimercaptobenzène, le 1,3,5-trimercaptobenzène, le 1,2,3-tris (mercaptométhyl)benzène, le 1,2,4-tris(mercaptométhyl)benzène, le 1,3,5-tris(mercaptométhyl)benzène, le 1,2,3-tris(mercaptoéthyl)benzène, le 1,2,4-tris(mercaptoéthyl)benzène, le 1,3,5-tris(mercaptoéthyl)benzène, le 1,2,3-tris(mercaptométhylèneoxy)benzène, le 1,2,4-tris (mercaptométhylèneoxy)benzène, le 1,3,5-tris(mercaptométhylèneoxy)benzène, le 1,2,3-tris(mercaptoéthylèneoxy)benzène, le 1,2,4-tris(mercaptoéthylèneoxy)benzène, le 1,3,5-tris(mercaptoéthylèneoxy)benzène, le 1,2,3,4-tétramercaptobenzène, le 1,2,3,5-tétramercaptobenzène, le 1,2,4,5-tétramercaptobenzène, le 1,2,3,4-tétrakis(mercaptométhyl)benzène, le 1,2,3,5-tétrakis(mercaptométhylbenzène), le 1,2,4,5-tétrakis(mercaptométhyl) benzène, le 1,2,3,4-tétrakis(mercaptoéthyl)benzène, le 1,2,3,5-tétrakis(mercaptoéthyl)benzène, le 1,2,4,5-tétrakis (mercaptoéthyl)-benzène, le 1,2,3,4-tétrakis(mercaptométhylèneoxy)-benzène, le 1,2,3,5-tétrakis(mercaptométhylèneoxy)-benzène, le 1,2,4,5-tétrakis(mercaptométhylèneoxy)-benzène, le 1,2,3,4-tétrakis(mercaptoéthylèneoxy)-benzène, le 1,2,3,5-tétrakis(mercaptoéthylèneoxy)-benzène, le 1,2,4,5-tétrakis(mercaptoéthylèneoxy)-benzène, le 2,2'-dimercaptobiphényle, 4,4'-dimercaptobiphényle, le 4,4'-dimercaptodibenzyle, le 2,5-toluènedithiol, le 3,4-toluènedithiol, le 1,4-naphtalènedithiol, le 1,5-naphtalènedithiol, le 2,6-naphtalènedithiol, le 2,7-naphtalènedithiol, le 2,4-diméthylbenzène-1,3-dithiol, le 4,5-diméthylbenzène-1,3-dithiol, le 9,10-anthracènediméthanethiol, le 1,3-di(p-méthoxyphényl)-propane-2,2-dithiol, le 1,3-diphénylpropane-2,2-dithiol, le phénylméthane-1,1-dithiol et le 2,4-di(p-mercaptophényl)pentane ;

(3) les polythiols aromatiques halogénés comprenant les polythiols aromatiques chlorés et bromés, tels que le 2,5-dichlorobenzène-1,3-dithiol, le 1,3-di-(p-chlorophényl)propane-2,2-dithiol, le 3,4,5-tribromo-1,2-dimercaptobenzène et le 2,3,4,6-tétrachloro-1,5-bis(mercaptométhyl)benzène ;

(4) les polythiols hétérocycliques, tels que la 2-méthylamino-4, 6-dithiol-sym-triazine, la 2-éthylamino-4,6-dithiol-sym-triazine, la 2-amino-4,6-dithiol-sym-triazine, la 2-morpholino-4,6-dithiol-sym-triazine, la 2-cyclohexylamino-4,6-dithiol-sym-triazine, la 2-méthoxy-4,6-dithiol-sym-triazine, la 2-phénoxy-4,6-dithiol-sym-triazine, la 2-thiobenzènoxy-4,6-dithiolsym-triazine, la 2-thiobutyloxy-4,6-dithiol-sym-triazine et la 2-thiobutyloxy-4,6-dithiol-sym-triazine, et ces mêmes composés substitués par halogène, tel que le chlore et le brome ;

(5) les polythiols ayant au moins un atome de soufre en plus des groupes mercapto et comprenant :

(5a) les polythiols aromatiques, tels que le 1,2-bis(mercaptométhylthio)benzène, le 1,3-bis(mercaptométhylthio)benzène, le 1,4-bis(mercaptométhylthio)benzène, le 1,2-bis(mercaptoéthylthio)benzène, le 1,3-bis(mercaptoéthylthio)benzène, le 1,4-bis(mercaptoéthylthio)benzène, le 1,2,3-tris(mercaptométhylthio)benzène, le 1,2,4-tris(mercaptométhylthio)benzène, le 1,3,5-tris-(mercaptométhylthio)benzène, le 1,2,3-tris(mercaptoéthylthio)benzène, le 1,2,4-tris(mercaptoéthylthio)benzène, le 1,3,5-tris(mercaptoéthylthio)benzène, le 1,2,3,4-tétrakis(mercaptométhylthio)benzène, le 1,2,3,5tétrakis(mercaptométhylthio)-benzène, le 1,2,4,5-tétrakis(mercaptométhylthio)benzène, le 1,2,3,4-tétrakis(mercaptoéthylthio)benzène, le 1,2,3,5-tétrakis(mercaptoéthylthio)benzène, le 1,2,4,5-tétrakis-(mercaptoéthyithio)benzène et les dérivés alkylés sur le cycle aromatique de ces polythiols ; et le 4,4'-thiodibenzènethiol ;

(5b) les polythiols aliphatiques, tels que le sulfure de bis(mercaptométhyle), le sulfure de bis(2-mercaptoéthyle), le sulfure de bis(2-mercaptopropyle), le bis(mercaptométhylthio)méthane, le bis(2-mercaptoéthylthio)méthane, le bis(3-mercaptopropylthio)-méthane, le tétrakis-(7-mercapto-2,5-dithiaheptyl)méthane, le 1,2-bis (mercaptométhylthio)-éthane, le 1,2-bis(2-mercaptoéthylthio)-éthane, le 1,2-bis(3-mercaptopropylthio)-éthane, le 1,3-bis(mercaptométhylthio)-propane, le 1,2-bis(2-mercaptoéthylthio)-propane, le 1,2-bis (3-mercaptopropylthio)-propane, le 1,2,3-tris(mercaptométhylthio)-propane, le 1,2,3-tris(2-mercaptoéthylthio)-propane, le 1,2,3-tris(3-mercaptopropyl-thio)propane, le tétrakis(mercaptométhyl-thiométhyl)méthane, le tétrakis(2-mercaptoéthylthiométhyl)méthane, le tétrakis(3-mercaptopropylthiométhyl)méthane, le sulfure de bis(2,3-dimercaptopropyle), le 2,5-dimercapto-1,4-dithiane, le disulfure de bis(mercaptométhyle), le disulfure de bis(mercaptoéthyle) et le disulfure de bis(mercaptopropyle), les esters de l'acide thioglycolique et de l'acide mercaptopropionique avec ces composés, le sulfure d'hydroxyméthyle bis (2-mercaptoacétate), le sulfure d'hydroxyméthyle bis (3-mercaptopropionate), le sulfure d'hydroxyéthyle bis (2-mercaptoacétate), le sulfure d'hydroxyéthyle bis(3-mercaptopropionate), le sulfure d'hydroxypropyle bis(2-mercaptoacétate), le sulfure d'hydroxypropyle bis(3-mercaptopropionate), le disulfure d'hydroxyméthyle bis(2-mercaptoacétate), le disulfure d'hydroxyméthyle bis (3-mercaptopropionate), le disulfure d'hydroxyéthyle bis(2-mercaptoacétate), le disulfure d'hydroxyéthyle bis(3-mercaptopropionate), le disulfure d'hydroxypropyle bis(2-mercaptoacétate), le disulfure d'hydroxypropyle bis(3-mercaptopropionate), l'éther 2-mercaptoéthylique bis(2-mercaptoacétate), l'éther

2-mercaptoéthylique bis(3-mercaptopropionate), le 1,4-dithian-2,5-diol bis(3-mercaptopropionate), l'acid thioglycolique bis(2-mercaptoéthyl ester), l'acide thiodipropionique bis(2-mercaptoéthyl ester), l'acide 4,4-thiodibutyrique bis(2-mercaptoéthyl ester), l'acide dithiodiglycolique bis(2-mercaptoéthyl ester), l'acide dithiodipropionique bis(2-mercaptoéthyl ester), l'acide 4,4-dithiodibutyrique bis(2-mercaptoéthyl ester), l'acide thiodiglycolique bis(2,3-dimercaptopropyl ester), l'acide thiodipropionique bis(2,3-dimercaptopropyl ester), l'acide dithiodiglycolique bis(2,3-dimercaptopropyl ester) et l'acide dithiodipropionique bis(2,3-dimercaptopropyl ester), et ces mêmes composés substitués par halogène, tel que le chlore et le brome ; le 4-mercaptométhyl-3,6-dithia-1,8-octanedithiol ; et

(5c) les composés hétérocycliques tels que le 3,4-thiophènedithiol, le 2,5-bis(mercaptométhyl)tétrahydrothiophène, le bis(mercaptométhyl)-1,3-dithiolane, le 2,5-dimercapto-1,3,4-diazole, le 2,5-dimercapto-1,4-dithiane et le 2,5-dimercaptométhyl-1,4-dithiane, et ces mêmes composés substitués par halogène tel que le chlore ou le brome ; et le tris(3-mercaptopropyl)isocyanurate ; et

(6) les produits de réaction de polythiols avec des composés polyinsaturés (excès de SH)/insaturations), tels que les produits d'addition 5 - éthylidène-2-norbonène- ou dicyclopentadiène - pentaérythritol tétrakis (mercapto acétate).

La présente invention a également pour objet un procédé de fabrication d'un produit soufré tel que défini ci-dessus, caractérisé par le fait que l'on fait réagir, à une température de 30 à 170°C, un mélange d'au moins un polythiol (I) et d'au moins un composé (II) tels que définis ci-dessus, les proportions des composés (I) et (II) étant choisies de telle sorte qu'il y ait ou qu'il y ait sensiblement un groupement thiol du ou des composés (I) pour une mole du ou des composés (II), afin d'obtenir le produit réticulable (P), et que, pour obtenir le produit (P'), on fait réagir un mélange du produit (P) ainsi obtenu et d'au moins un composé de formule (I), dans des proportions stoechiométriques ou sensiblement stoechiométriques entre les groupements thiol et les doubles liaisons, par voie radicalaire, les radicaux pouvant être générés par voie thermique et/ou par voie d'irradiation UV ou visible ou par voie de faisceau d'électrons.

On peut conduire la réaction des composés (I) et (II) en présence d'au moins un catalyseur choisi notamment parmi les initiateurs de radicaux libres tels que les peroxydes comme le peroxyde de benzoyle, le peroxyde de p-chlorobenzyle et autres peroxydes de di-acyle, le peroxyde de di-tert.-butyle ; les peroxy esters comme le peroxy-2-éthyl hexanoate de t-butyle, le peroxy néodécanoate de t-butyle ; les peroxy dicarbonates comme le peroxy dicarbonate de diisopropyle, le peroxy dicarbonate de di-2-éthylhexyle ; et les composés azoïques, comme l'azobisisobutyronitrile ; et parmi les acides de Lewis comme le tétrafluorure acide de bore, le trifluorure acide de bore et les complexes de coordination de ces derniers avec l'eau, les esters, les aldéhydes, les alcools, les acides, les cétones ou les éthers, tels que $BF_3 \cdot (H_2O)_x$ (x = 1 ou 2), $BF_3 \cdot C_4H_9OH$, $BF_3 \cdot 2CH_3COOH$, $BF_3 \cdot 2CH_2COOC_2H_5$, $BF_3 \cdot CH_3COCH_3$, $BF_3 \cdot O(C_2H_5)_2$ ou $BF_3 \cdot C_4H_9OC_4H_9$ ; l'acide chlorhydrique, l'acide sulfurique, les esters de l'acide sulfurique comme l'acide éthyl sulfurique, les acides sulfoniques organiques comme l'acide toluène sulfonique ou l'acide butyl sulfonique ; et les amines comme la triéthylamine. Le catalyseur peut être utilisé par exemple, pour une fonction thiol du polythiol (I), à raison de 0,0001 à 0,5 mole dans le cas des peroxydes, des peroxy esters, des peroxy carbonates et des composés azoïques, de 0,01 à 2,0 moles dans le cas des acides et acides de Lewis, de 0,05 à 0,1 mole dans le cas des amines.

La réaction des composés (I) et (II) peut être conduite en l'absence de solvant ou en présence d'un solvant, qui peut être notamment un solvant aromatique, aliphatique ou cycloaliphatique, ledit solvant étant éliminé en fin de réaction.

La réaction du ou des polythiols (I) sur le produit (P) peut être conduite par voie photochimique, auquel cas on peut opérer en l'absence d'un photoinitiateur ou système photoinitiateur, ou en présence d'au moins un photoinitiateur ou d'un système photoinitiateur, auquel peut être associé un agent photoactivateur. Il est également possible d'opérer par voie d'irradiation UV, sans photoinitiateur.

Le photoinitiateur utilisé selon l'invention peut être tout composé capable de générer des radicaux libres sous l'effet du rayonnement ultraviolet.

On peut citer à titre d'exemples :

- les $\alpha$-dicétones, comme le benzile et le diacétyle ;
- les acyloïnes, comme la benzoïne ;
- les acyloïn éthers, comme le benzoïn méthyl éther, le benzoin éthyl éther, le benzoïn isopropyl éther et le benzoïn isobutyl éther ;
- les thioxanthones, comme la thioxanthone, la 2,4-diéthylthioxanthone, l'acide thioxanthone-1-sulfonique, l'isopropyl-thioxanthone-4 sulfonique, l'isopropylthioxanthone et la 2-chlorothioxanthone ;
- les benzophénones, comme 1a benzophénone, la 4,4-bis(diméthylamino)benzophénone, la 4,4'-bis(diéthylamino)benzophénone, la 4,4'-diéthylaminobenzophénone, la cétone de Mischler ;

- les propiophénones, comme la 2-hydroxy-2-méthylpropiophénone, la 4'-isopropyl-2-hydroxy-2-méthyl-propiophénone ;
- les acétophénones, comme l'acétophénone, la p-diméthylaminoacétophénone, l'α,α'-diméthoxyacétoxyacétophénone, la 2,2-diméthoxy-2-phénylacétophénone, la p-méthoxyacétophénone, la 2-méthyl-[4-(méthylthio)phényl]-2-morpholino-1-propanone, la 2,2-diéthoxyacétophénone, la 4'-phénoxy-2,2-dichloroacétophénone, la 2-benzyl-2-N,N-diméthylamino-1-(4-morpholinophényl)-butanone-1, la 2,2-diméthoxy-2-phényl-acétophénone, la 2-hydroxy-2-méthyl-1-phénylpropanone;
- les quinones, comme l'anthraquinone la 2-éthylanthraquinone, la 2-chloroanthraquinone, la 1,4-naphtoquinone ;
- les alpha-hydroxyarylcétones, comme la 1-hydroxycyclohexyl phényl cétone ;
- les composés halogénés, tels que le chlorure de phénacyle, la tribromométhylphénylsulfone, la tris(trichlorométhyl)-s-triadine ;
- les monoacyl phosphine oxydes, tels que le 2,4,6-triméthyl benzoyldiphényl phosphine oxyde et le 2,4,6-triméthyl benzoyl éthoxyphényl phosphine oxyde ;
- les bis acyl phosphine oxydes, tels que le bis(2,6-dichlorobenzoyl)-(4-propylphényl)phosphine oxyde, le bis(2,6-diméthoxybenzoyl)-2,4,4-triméthyl pentyl phosphine oxyde, le bis(2,4,6-triméthylbenzoyl)-phényl phosphine oxyde et le bis(2,4,6-triméthylbenzoyl)-2,4,4-triméthylpentyl phosphine oxyde ;
- les α-sulfocétones ; et
- d'autres composés comme le benzile diméthyl cétal ; le. N,N-diméthylaminobenzoate d'isoamyle, le N,N-diméthylaminobenzoate d'éthyle, le benzoïn benzoate, la 2-hydroxy-2-méthyl-1-phényl propanone, l'α-acyloxime ester.

Lorsqu'un photoinitiateur est utilisé, les composés ci-dessus peuvent être utilisés soit individuellement en tant que photoinitiateur, soit sous la forme d'un mélange d'au moins deux d'entre eux en tant que système photoinitiateur. Par ailleurs, à l'initiateur ou au système photoinitiateur, peut être associé au moins un agent photoactivateur.

La proportion de photoinitiateur ou de système photoinitiateur selon l'invention est par exemple comprise entre 0,5 et 10% en poids environ de préférence entre 1 et 5% en poids environ, par rapport à la masse de la formulation du ou des produits (P) avec le ou les polythiols.

La réaction du ou des polythiols (I) sur le produit (P) peut aussi être conduite par voie thermique, auquel cas on opère avantageusement en présence d'au moins un amorceur, producteur de radicaux libres, auquel peut être associé un accélérateur.

L'amorceur est notamment constitué par au moins l'un parmi un peroxyde organique tel que le peroxyde de benzoyle, le 2,5-diméthyl-2,5-bis (2-éthylhexylperoxy) hexane, le peroxyde de méthyl éthyl cétone, le peroxyde de 2,4-pentanedione ; un peroxydicarbonate ; un peroxyester tel que le peroxybenzoate de tertiobutyle, le peroxyoctoate de tertiobutyle, le peroxyoctoate de tertioamyle ou le 2,5-diperoxyoctoate. Cet amorceur est utilisé notamment à raison de 0,5 à 3% en poids par rapport à la masse de la formulation du ou des produits (P) avec le ou les polythiols.

Comme accélérateur de réticulation par voie thermique, utilisé de préférence en proportion de 0,1 à 1% en poids par rapport à la masse de la formulation du ou des produits (P) avec le ou les polythiols, on peut citer notamment des solutions, dans un solvant organique, de dioctyl phtalate, de sels minéraux ou organiques de métal de transition tel que vanadium, chrome, manganèse, fer, cobalt, nickel, cuivre, zinc, molybdène et plomb, ou encore des amines tertiaires telles que la diméthylaniline ou la N,N-diméthylparatoluidine.

Lorsque le peroxyde amorceur est le peroxyde de benzoyle, on préfère utiliser une amine tertiaire comme accélérateur. Lorsque le peroxyde amorceur est le peroxyde de méthyl éthyl cétone, on préfère utiliser comme accélérateur un sel tel que le naphténate ou l'octoate de cobalt.

On peut également utiliser simultanément un photoinitiateur ou système photoinitiateur et, le cas échéant, un agent photoactivateur, et un amorceur de réticulation par voie thermique avec, le cas échéant un accélérateur.

On peut déposer le mélange réactionnel du ou des polythiols (I) et du produit de réaction (P) en couche mince sur un substrat et conduire la réaction par voie radicalaire afin d'obtenir le produit réticulé (P') sous forme de film ; on peut également introduire le mélange réactionnel du ou des polythiols (I) et du produit de réaction (P) dans un moule et conduire la réaction par voie radicalaire afin d'obtenir le produit réticulé (P') sous la forme d'une pièce moulée, le moule étant un moule transparent à au moins une partie des rayonnements de longueurs d'onde comprises entre 180 et 400 nm dans le cas où l'on utilise la voie photochimique.

La présente invention concerne également une composition réticulable par voie radicalaire, les radicaux pouvant être générés par voie thermique et/ou par voie d'irradiation UV ou visible ou par voie de faisceau d'électrons, caractérisée par le fait qu'elle comprend au moins un produit réticulable (P) tel que défini ci-dessus.

En particulier, ladite composition comprend :

(a) au moins un produit réticulable (P) tel que défini ci-dessus ;

(b) au moins un agent réticulant consistant en au moins un polythiol (I) tel que défini ci-dessus, qui peut ou peuvent

être différent(s) du ou des polythiols utilisés pour la préparation dudit produit (P) ;
(c) en cas de réticulation par voie photochimique, le cas échéant un photoinitiateur ou un système photoinitiateur, auquel peut être associé un agent photoactivateur ;
(d) en cas de réticulation par voie thermique, un amorceur producteur de radicaux libres, auquel peut être associé un accélérateur ;
(e) le cas échéant, au moins un diluant réactif monomère ou oligomère ;
(f) le cas échéant, au moins un solvant ou diluant non réactif ; et
(g) le cas échéant au moins un additif usuel, tel qu'un pigment.

Le photoinitiateur ou système photoinitiateur est choisi notamment parmi ceux décrits ci-dessus et utilisé généralement à raison de 1 à 5% en poids par rapport à la masse de la formulation du ou des produits (P) avec le ou les polythiols.

L'amorceur de voie thermique et l'accélérateur associé sont également choisis parmi ceux décrits ci-dessus et utilisés généralement à raison de respectivement 0,5 à 3% en poids et 0,1 à 1% en poids par rapport à la masse de la formulation du ou des produits (P) avec le ou les polythiols.

Les compositions selon l'invention peuvent en outre comprendre au moins l'un parmi un diluant réactif monomère ou oligomère, un solvant ou diluant non réactif, et un additif usuel tel qu'un pigment.

Comme diluant réactif monomère ou oligomère, on peut citer des monomères vinyliques, tels que l'acétate de vinyle, le styrène, le vinyl toluène, le divinyl benzène ; des esters acryliques et méthacryliques, tels que le (méth)acrylate de méthyle, le (méth)acrylate d'éthyle, le (méth)acrylate d'isopropyle, le (méth)acrylate de n-butyle, le (méth)acrylate d'isobutyle, le (méth)acrylate de 2-éthylhexyle, le (méth)acrylate de 2-hydroxyéthyle, le (méth)acrylate de glycidyle, le di(méth)acrylate d'éthylène glycol, le (di)méthacrylate de diéthylène glycol, le di(méth)acrylate de tétraéthylène glycol, le di(méth)acrylate de glycérol, le tri(méth)acrylate de glycérol, le di(méth)acrylate de 1,3-propylène glycol, le di(méth)acrylate de dipropylène glycol, le di(méth)acrylate de tripropylène glycol, le di(méth)acrylate de 1,4-butanediol, le tri(méth)acrylate de 1,2,4-butanetriol, le di(méth)acrylate de 1,6-hexanediol, le di(méth)acrylate de 1,4-cyclohexanediol, le di(méth)acrylate de 1,4-benzènediol, le tétra(méth)acrylate de pentaérythritol, le di(méth)acrylate de 1,5-pentanediol, le di(méth)acrylate de triméthylolpropane, le tri(méth)acrylate de triméthylolpropane, le 2,2-diméthyl-3-hydroxypropyl-2,2-diméthyl-3-hydroxypropionate, le (méth)acrylate d'isobornyle et le (méth)acrylate de tétrahydrofurfuryle ; des (méth)acrylates issus de glycidyl éthers aromatiques tels que le bisphénol A - diglycidyl éther et de glycidyl éthers aliphatiques tels que le butanediol diglycidyl éther, des exemples spécifiques de ceux-ci comprenant le 1,4-butanediol diglycidyléther di(méth)acrylate, le bisphénol A - diglycidyléther di(méth)acrylate et le néopentylglycol diglycidyléther di(méth)acrylate ; et des amides acryliques ou méthacryliques tels que le (méth)acrylamide, le diacétone (méth)acrylamide, le N(bêta-hydroxyéthyl) (méth)acrylamide, le N,N-bis(bêta-hydroxyéthyl) (méth)acrylamide, le méthylène bis(méth)acrylamide, le 1,6-hexaméthylène bis(méth)acrylamide, le diéthylènetriamine tris(méth)acrylamide, le bis (gamma- (méth) acrylamidepropoxy) éthane et le bêta-(méth)acrylamide éthylacrylate.

Comme solvant ou diluant non réactif, on peut citer l'acétate d'éthyle, l'acétate de butyle, le méthoxypropanol, l'isopropanol, la méthyl éthyl cétone, l'acétone.

Les pigments sont notamment le bleu de phtalocyanine et le dioxyde de titane.

On peut également placer la composition sous la forme d'une émulsion dans l'eau ou d'une dispersion dans l'eau.

Si on le désire, les compositions de la présente invention sont stabilisées. La stabilisation de la formulation est réalisée à l'aide d'un ou de plusieurs inhibiteurs de polymérisation radicalaire mis en oeuvre dans les quantités classiques et choisis notamment parmi les inhibiteurs de type phénolique, les quinones, la phénothiazine et ses dérivés, les composés porteurs de radicaux nitroxyles, les amines à encombrement stérique, les composés nitro aromatiques, les composés à fonction nitroso ou N-nitroso, les phosphites, les thioéthers. Comme exemples d'inhibiteurs de type phénolique, on peut citer entre autres l'hydroquinone, l'éther monométhylique de l'hydroquinone, l'ortho tertiobutyl phénol, le 2,6-ditertiobutylphénol, le 2,6-ditertiobutyl paranitroso phénol, le paracrésol, le 2,6-ditertiobutyl paracrésol et le 2,6-ditertiobutyl paraméthoxy phénol.

La présente invention porte également sur les produits obtenus par la réticulation par voie radicalaire de la composition telle que définie ci-dessus, lesdits produits se présentant sous la forme de films, de films revêtant un substrat, ou de pièces moulées.

Pour l'obtention de films, on dépose la composition en couche mince sur un substrat et on conduit la réticulation de celle-ci par voie radicalaire. Les compositions de revêtement selon l'invention peuvent être appliquées sur les substrats les plus divers : bois, papier, panneaux d'agglomérés, panneaux de particules, métaux, métaux revêtus d'un primaire, verre, matières plastiques, matières plastiques métallisées. L'application du film de composition sur le substrat peut être effectuée par immersion, pulvérisation, revêtement au rouleau, machine à rideau, etc.

Pour l'obtention de pièces moulées, on introduit la composition dans un moule et on conduit la réticulation de celle-ci par voie radicalaire, le moule étant un moule transparent à au moins une partie des rayonnements de

longueurs d'onde comprises entre 180 et 400 nm dans le cas où l'on utilise la voie photochimique.

Lorsque les compositions selon l'invention sont destinées à être durcies par la lumière ultraviolette, on utilise n'importe quelle source appropriée qui émet une lumière ultraviolette ayant une longueur d'onde de 180-400 nm, telle que arcs de mercure, arcs de carbone, lampes à mercure basse pression ou moyenne pression ou haute pression, arcs à plasma à écoulement rotationnel et diodes électroluminescentes à lumière ultraviolette. La puissance de ces lampes à longs tubes est de l'ordre de 80-240 Watts par cm de longueur de tube. Généralement, on fait se déplacer le substrat revêtu d'un film mince de la composition au-dessous d'une ou plusieurs des lampes précitées, à une vitesse comprise entre 1 et 300 mètres par minute.

Les Exemples suivants illustrent la présente invention sans toutefois en limiter la portée. Dans ces Exemples, les pourcentages sont en poids sauf indication contraire.

EXEMPLE DE SYNTHÈSE 1

Dans un réacteur muni d'un condenseur, d'une agitation magnétique et d'une alimentation d'azote permettant de travailler sous atmosphère inerte, on introduit 12,6 g de 5-vinyl-2-norbornène (pureté : 95%). On chauffe à 70°C et on additionne 8,5 g de sulfure de bis-(2-mercaptoéthyle) en l'espace de 30 minutes à l'aide d'un ampoule à addition.

Au cours de l'addition, on observe une exothermie et la température du milieu réactionnel atteint 165°C. La réaction est suivie par infrarouge jusqu'à disparition totale de la bande -S-H à 2 571 $cm^{-1}$.

Le produit obtenu présente un indice de réfraction de 1,571 à 25°C.

EXEMPLE DE SYNTHÈSE 2

On opère de la même manière qu'à l'Exemple de synthèse 1 en utilisant 11,2 g de 4-mercaptométhyl-3,6-dithia-1,8-octane dithiol (pureté : 93%) et 15,2 g de 5-vinyl-2-norbornène.

La température maximale atteinte au cours de l'addition du thiol est de 79°C. Le produit obtenu présente un indice de réfraction de 1,579 à 25°C.

EXEMPLE DE SYNTHÈSE 3

On opère de la même manière qu'à l'Exemple de synthèse 1 en utilisant 12,2 g de pentaérythritol tétrakis (3-mercapto propionate) et 12,6 g de 5-vinyl-2-norbornène.

La température maximale atteinte au cours de l'addition du thiol est de 134°C. Le produit obtenu présente une viscosité de 0,48 Pa.s à 25°C et un indice de réfraction de 1,531 à 25°C.

EXEMPLE DE SYNTHÈSE 4

On opère de la même manière qu'à l'Exemple de synthèse 1 en utilisant 12,2 g de pentaérythritol tétrakis (3-mercapto propionate) et 12 g de 5-éthylidène-2-norbornène (pureté : 99%).

La température maximale atteinte lors de l'addition du thiol est de 95°C. Le produit obtenu présente une viscosité de 7,66 Pa.s à 25°C et un indice de réfraction de 1,5445 à 25°C.

EXEMPLE DE SYNTHÈSE 5

Dans l'équipeme décrit à l'Exemple de synthèse 1, on introduit 24 g de 5-éthylidène-2-norbornène qu'on porte à 116°C. Lorsque la température est de 116°C, on lance l'addition continue de 24,4 g de pentaérythritol tétrakis (3-mercapto propionate) sur une durée de 35 min. (41,83 g/h). On maintient la température à 116°C pendant les 5 premières minutes d'addition, puis on diminue la température jusqu'à 84°C sur une période de 30 minutes (rampe de température de - 1,07°C/min). Lorsque l'addition est terminée, on laisse revenir à température ambiante. L'absence de bande -S-H à 2 571 $cm^{-1}$ indique que la totalité du dithiol a été consommée. Le produit obtenu présente un indice de réfraction de 1,5370 à 26°C et une viscosité de 1,16 Pa.s à 25°C.

EXEMPLE DE SYNTHÈSE 6

Dans l'équipement décrit à l'Exemple de synthèse 1, on introduit 50,1 g de 5-vinyl-2-norbornène qu'on porte à 118°C. Lorsque la température est de 118°C, on lance l'addition continue de 31,1 g de sulfure de 2-mercaptoéthyle sur une durée de 2 heures (15,55 g/h). On maintient la température à 118°C pendant les 10 premières minutes d'addition, puis on diminue la température jusqu'à 82°C sur une période d'une heure (rampe de température de - 0,6°C/min.). On maintient une température de 82°C jusqu'à la fin de l'addition du dithiol puis on laisse revenir à température

ambiante. L'absence de bande -S-H à 2 571 $cm^{-1}$ indique que la totalité du dithiol a été consommée. Le produit obtenu présente un indice de réfraction de 1,5610 à 24°C et une viscosité de 0,06 Pa.s à 25°C.

EXEMPLE DE SYNTHÈSE 7

On opère de la même manière qu'à l'Exemple de synthèse 6 en utilisant 50,9 g de 5-vinyl-2-norbornène et 37,5 g de 4-mercaptométhyl-3,6-dithia-1,8-octane dithiol (débit d'addition de 18,75 g/h). Le produit obtenu présente un indice de réfraction de 1,5725 à 23°C et une viscosité de 0,2 Pa.s à 25°C.

EXEMPLE DE SYNTHÈSE 8

On opère de la même manière qu'à l'Exemple de synthèse 6 en utilisant 38,9 g de 5-vinyl-2-norbornène et 37,5 g de 4-pentaérythritol tétrakis (3-mercaptopropionate)(débit d'addition de 18,75 g/h). Le produit obtenu présente un indice de réfraction de 1,5275 à 26°C et une viscosité de 0,27 Pa.s à 25°C.

Dans tous les exemples d'application qui suivent, sauf indication contraire, les formulations sont appliquées sur verre à l'aide d'une barre "K hand coater" de manière à obtenir un film d'une épaisseur de 12 μm. Lorsque le durcissement est effectué par la lumière ultraviolette, la lampe utilisée est une lampe F450 (H-bulb) de puissance 120 Watts/cm commercialisée par la Société FUSION, et les photoinitiateurs utilisés sont le Darocure® 1173 (2-hydroxy-2-méthyl-1-phényl-propanone-1) et l'Irgacure® 184 (1-hydroxy-cyclohexyl phényl cétone). La dureté est mesurée après 24 h à température ambiante.

EXEMPLE D'APPLICATION 1

A 61,75 g du produit obtenu à l'Exemple de synthèse 1, on ajoute 38,25 g de pentaérythritol tétrakis (3-mercaptopropionate). L'indice de réfraction de la formulation obtenue est de 1,564 à 25°C. Un film présentant une dureté crayon de 2H est obtenu après 2 passages sous la lampe à la vitesse de 5 mètres/min. Lorsqu'on ajoute 1 g de Darocure® 1173 et 1 g d'Irgacure® 184, on obtient un film sec présentant une dureté crayon de 4H après 2 passages sous la lampe à 54 mètres/min.

EXEMPLE D'APPLICATION 2

La formulation décrite ci-dessus contenant 1 g de Darocure® 1173 et 1 g d'Irgacure® 184 est déposée dans un verre de montre (épaisseur maximale de 3 mm). Après 5 passages sous la lampe à la vitesse de 5 mètres/min, on obtient un matériau réticulé transparente et souple.

EXEMPLE D'APPLICATION 3

A la formulation décrite à l'Exemple d'application 2, on ajoute 0,5 g d'octoate de cobalt et 1,5 g de peroxyde de méthyl éthyl cétone. La formulation est moulée dans un verre de montre. Après 5 passages sous la lampe à la vitesse de 5 mètres/min. et 30 min. à 130°C, on obtient un matériau réticulé souple.

EXEMPLE D'APPLICATION 4

A 37,05 g du produit obtenu à l'Exemple de synthèse 1, on ajoute 22,95 g de pentaérythritol tétrakis (3-mercaptopropionate), 40 g de triacrylate de triméthylolpropane (commercialisé par la Société Sartomer sous la dénomination SR 351), 1 g de Darocure® 1173 et 1 g d'Irgacure® 184.

Un film sec présentant une dureté crayon de 2H est obtenu après 1 passage sous la lampe à la vitesse de 54 mètres/min.

EXEMPLE D'APPLICATION 5

La formulation décrite ci-dessus est coulée dans un verre de montre. Après 5 passages sous la lampe à 5 mètres/min., on obtient un matériau réticulé, transparent et dur.

EXEMPLE D'APPLICATION 6

A 62,85 g du produit obtenu à l'Exemple de synthèse 2, on ajoute 37,15 g de pentaérythritol tétrakis (3-mercapto propionate), 1 g de Darocure® 1173 et 1 g d'Irgacure® 184. L'indice de réfraction de la formulation est de 1,565

à 25°C. Un film sec présentant une dureté de H est obtenu après 3 passages à la vitesse de 54 mètres/min.

EXEMPLE D'APPLICATION 7

La formulation de l'Exemple d'application 6 est coulée dans un verre de montre. Après 5 passages à la vitesse de 5 mètres/min., on obtient un matériau réticulé, opaque et souple.

EXEMPLE D'APPLICATION 8

A 70,45 g du produit obtenu à l'Exemple de synthèse 2, on ajoute 29,55 g de 4-mercaptométhyl-3,6-dithia-1,8-octanedithiol, 1 g de Darocure® 1173 et 1 g d'Irgacure® 184. La formulation, qui présente un indice de réfraction de 1,594 à 25°C, est coulée dans un verre de montre. Après 5 passages à la vitesse de 5 mètres/min., on obtient un matériau réticulé, transparent et souple.

EXEMPLE D'APPLICATION 9

A 66,5 g du produit obtenu à l'Exemple de synthèse 2, on ajoute 13,9 g de 4-mercaptométhyl-3,6-dithia-1,8-octanedithiol, 19,6 g de pentaérythritol tétrakis (3-mercaptopropionate), 1 g de Darocure® 1173 et 1 g d'Irgacure® 184. L'indice de réfraction de la formulation obtenue est de 1,579 à 25°C. Un film sec présentant une dureté crayon de H est obtenu après 1 passage sous la lampe à la vitesse de 54 mètres/min.

EXEMPLE D'APPLICATION 10

La formulation de l'Exemple d'application 9 est coulée dans un verre de montre. Après 8 passages à la vitesse de 5 mètres/min., on obtient un matériau réticulé, transparent et souple.

EXEMPLE D'APPLICATION 11

A 66,5 g du produit obtenu à l'Exemple de synthèse 3, on ajoute 33,5 g de pentaérythritol tétrakis (3-mercaptopropionate). L'indice de réfraction de la formulation obtenue est de 1,531 à 25°C. Un film présentant une dureté crayon de H est obtenu après 1 passage sous la lampe à la vitesse de 54 mètres/min.

EXEMPLE D'APPLICATION 12

A la formulation de l'Exemple d'application 11, on ajoute 1 g de Darocure® 1173 et 1 g d'Irgacure® 184. La formulation est coulée dans un verre de montre. Après 5 passages sous la lampe à la vitesse de 5 mètres/min., on obtient un matériau réticulé, transparent et dur.

EXEMPLE D'APPLICATION 13

A 73,65 g du produit obtenu à l'Exemple de synthèse 3, on ajoute 26,35 g de 4-mercaptométhyl-3,6-dithia-1,8-octane dithiol, 1 g de Darocure® 1173 et 1 g d'Irgacure® 184. La formulation, qui présente un indice de réfraction de 1,555 à 25°C, est coulée dans un verre de montre. Après 8 passages sous la lampe à la vitesse de 5 mètres/min., on obtient un matériau réticulé, transparent et souple.

EXEMPLE D'APPLICATION 14

A 66,5 g du produit obtenu à l'Exemple de synthèse 3, on ajoute 33,5 g de pentaérythritol tétrakis (3-mercaptopropionate), 1,5 g de peroxyde de méthyl éthyl cétone et 0,5 g d'octanoate de cobalt. La formulation est coulée dans un verre de montre. Après 1 heure à 60°C, on obtient un matériau réticulé, souple.

EXEMPLE D'APPLICATION 15

A 66,5 g du produit obtenu à l'Exemple de synthèse 4, on ajoute 33,5 g de pentaérythritol tétrakis (3-mercaptopropionate). L'indice de réfraction de la formulation obtenue est de 1,5395 à 25°C. Un film présentant une dureté de H est obtenu après 3 passages sous la lampe à la vitesse de 54 mètres/min.

EXEMPLE D'APPLICATION 16

A la formulation de l'Exemple d'application 15, on ajoute 1 g de Darocure® 1173 et 4 g d'Irgacure® 184. La formulation est coulée dans un verre de montre. Après 5 passages sous la lampe à la vitesse de 5 mètres/min., on obtient un matériau réticulé, transparent et dur.

EXEMPLES D'ESSAI 1 à 6 : propriétés mécaniques des films réticulés

Des films réticulés d'une épaisseur de 200 µm ont été préparés à partir de la formulation suivante :

| | |
|---|---|
| Résine + agent réticulant | 98 % |
| Darocure 1173 | 1 % |
| Irgacure 184 | 1 % |
| | 100 % |

La réticulation est effectuée par 3 passages à 5m/min. sous la lampe F450 (H-bulb) d'une puissance de 120 Watts/cm.

| Exemple d'essai | RÉSINE selon l'Exemple de Synthèse (quantité) | AGENT RÉTICULANT* (quantité) | Tα (1) (°C) | tanδ max (1) | Er** (1) (MPa) | HU (2) (MPa) | Contrainte à la rupture (MPa) (3) | Elongation (3) (%) | Module (3) (MPa) |
|---|---|---|---|---|---|---|---|---|---|
| 1(Film 1) | 1 (394,4 g) | Trithiol (173,3g) | 7 | 1,36 | 3,1 | 0,45 | 0,88 ± 0,7 | 42 ± 5 | 2,9 ± 0,1 |
| 2(Film 2) | 1 (394,4 g) | Tétrathiol (244,35g) | 19 | 1,04 | 5,8 | 0,81 | 1,8 ± 0,2 | 40 ± 5 | 5,8 ± 0,1 |
| 3(Film 3) | 2 (620,6 g) | Trithiol (260 g) | 19 | 1,25 | 6,9 | 1 | 1,7 ± 0,3 | 28 ± 5 | 7,2 ± 0,1 |
| 4(Film 4) | 3 (969,5 g) | Dithiol (308 g) | 6 | 1,46 | 4,9 | 0,7 | 1,5 ± 0,3 | 36 ± 6 | 5,3 ± 0,1 |
| 5(Film 5) | 3 (969,5 g) | Trithiol (346,67 g) | 28 | 0,94 | 8,6-11 | 1,8 | 4 ± 0,4 | 53 ± 5 | 13 ± 0,3 |
| 6(Film 6) | 3 (969,5 g) | Tétrathiol (488,7 g) | 31 | 1 | 11 | 7,8 | 10 ± 1 | 60±11 | 255 ± 33 |

* <u>Agent réticulant</u> :

Dithiol = Sulfure de bis-(2-mercaptoéthyle)
Trithiol = 4-mercaptométhyle-3,6-dithia-1,8-octane dithiol
Tétrathiol = Pentaérythritol tétrakis (3-mercapto propionate)

** Er = module au plateau caoutchoutique
(1) mesures effectuées sur RSAII de Rheometric Scientific à une fréquence de 1Hz
(2) mesure effectuée sur microduromètre H100 de Fisher Instrumentation Electronique
(3) moyenne de 5 mesures effectuées sur machine Instron (5m/min à 23°C)

**Revendications**

1. Produit soufré obtenu par la réaction d'addition d'au moins un polythiol (I) sur au moins un composé de formule (II) :

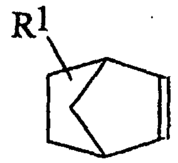

(II)

dans laquelle $R^1$ représente un reste aliphatique, linéaire ou ramifié, comportant une double liaison d'insaturation éthylénique, cette dernière pouvant être celle par laquelle $R^1$ est relié au cycle norbornène, ledit cycle pouvant également comporter d'autres substituants choisis parmi alkyle en $C_1$-$C_3$, dans des proportions correspondant à un groupement thiol du ou des polythiols (I) par mole du ou des composés (II), les groupes thiol du ou des composés (I) s'étant additionnés majoritairement sur la double liaison cyclique du ou des composés (II) et, en moyenne, une insaturation éthylénique d'un composé (II) sur les deux qu'il porte étant restée libre.

2. Produit soufré selon la revendication 1, **caractérisé par le fait que** le composé de formule (II) est le 5-vinyl-2-norbornène (5-vinylbicyclo[2.2.1]hepto-2-ène) ou le 5-éthylidène-2-norbornène (5-éthylidène-bicyclo[2.2.1] hepto-2-ène).

3. Produit soufré selon l'une des revendications 1 et 2, **caractérisé par le fait que** les polythiols (I) sont choisis parmi les composés de formule $R^2$-[-SH]$_n$ dans laquelle :

   - $R^2$ est un radical n-valent aliphatique, aromatique ou hétérocyclique ou comportant une combinaison de ceux-ci, $R^2$ pouvant comporter des substituants, le squelette ou les substituants pouvant être interrompus par au moins un hétéroatome choisi parmi -O- ou -S- et/ou un groupement choisi parmi

   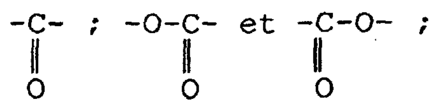

   et

   - n est un entier de 2 à 6.

4. Produit soufré selon la revendications 1, **caractérisé par le fait que** les polythiols (I) sont des polythiols obtenus par addition d'un polythiol de formule $R^2$-[-SH]$_n$ telle que définie à la revendication 3, sur un composé de formule (II) telle que définie à la revendication 1, avec un excès de thiol et dans des conditions d'addition évitant une réticulation.

5. Produit soufré selon la revendication 1, **caractérisé par le fait que** le polythiol (I) est choisi parmi le sulfure de dimercaptoéthyle, le 4-mercaptométhyl-3,6-dithia-1,8-octane dithiol, le pentaérythritol tétrakis(3-mercaptopropionate) et le pentaérythritol tétrakis(2-mercapto acétate).

6. Procédé de fabrication du produit soufré tel que défini à l'une des revendications 1 à 5, **caractérisé par le fait que** l'on fait réagir, à une température de 30 à 170°C, un mélange d'au moins un polythiol (I) et d'au moins un composé (II) tels que définis à l'une des revendications 1 à 5, les proportions des composés (I) et (II) étant choisies de telle sorte qu'il y ait un groupement thiol du ou des composés (I) pour une mole du ou des composés (II), afin d'obtenir le produit réticulable (P), et que, pour obtenir le produit (P'), on fait réagir un mélange du produit (P) ainsi obtenu et d'au moins un composé de formule (I), dans des proportions stoechiométriques entre les groupements thiol et les doubles liaisons, par voie radicalaire, les radicaux pouvant être générés par voie thermique et/ou par voie d'irradiation UV ou visible ou par voie de faisceau d'électrons.

**7.** Procédé selon la revendication 6, **caractérisé par le fait que** l'on conduit la réaction des composés (I) et (II) en présence d'au moins un catalyseur choisi parmi les initiateurs de radicaux libres, tels que les peroxydes, les peroxy esters, les peroxy dicarbonates et les composés azoïques, et les acides et acides de Lewis et les amines.

**8.** Procédé selon l'une des revendications 6 et 7, **caractérisé par le fait que** l'on conduit la réaction des composés (I) et (II) en l'absence de solvant.

**9.** Procédé selon l'une des revendications 6 à 8, **caractérisé par le fait que** l'on conduit la réaction des composés (I) et (II) en milieu solvant, le solvant étant éliminé en fin de réaction.

**10.** Utilisation du produit (P) tel que défini à l'une des revendications 1 à 5, dans une composition réticulable par voie radicalaire, contenant au moins un polythiol (I) dans des proportions stoechiométriques entre les groupements thiol du ou des polythiols (I) et les doubles liaisons dudit produit (P), les doubles liaisons dudit produit (P) étant destinées à être totalement consommées en vue d'obtenir un produit (P') réticulé.

**11.** Utilisation selon la revendication 10, **caractérisée par le fait que** l'on conduit la réaction du ou des polythiols (I) sur le produit (P) par voie photochimique en l'absence de photoinitiateur ou de système photoinitiateur, ou bien en présence d'au moins un photoinitiateur ou d'un système photoinitiateur, auquel peut être associé un agent photoactivateur.

**12.** Utilisation selon l'une des revendications 10 et 11, **caractérisée par le fait que** l'on conduit la réaction du ou des polythiols (I) sur le produit (P) par voie thermique, en présence d'au moins un amorceur, producteur de radicaux libres, auquel peut être associé un accélérateur.

**13.** Utilisation selon l'une des revendications 10 à 12, **caractérisée par le fait qu'**on dépose le mélange réactionnel du ou des polythiols (I) et du produit de réaction (P) en couche mince sur un substrat et qu'on conduit la réaction par voie radicalaire afin d'obtenir le produit réticulé (P') sous forme de film.

**14.** Utilisation selon l'une des revendications 10 à 12, **caractérisée par le fait qu'**on introduit le mélange réactionnel du ou des polythiols (I) et du produit de réaction (P) dans un moule et qu'on conduit la réaction par voie radicalaire afin d'obtenir le produit réticulé (P') sous la forme d'une pièce moulée, le moule étant un moule transparent à au moins une partie des rayonnements de longueurs d'onde comprises entre 180 et 400 nm dans le cas où l'on utilise la voie photochimique.

**15.** Composition réticulable par voie radicalaire, les radicaux pouvant être générés par voie thermique et/ou par voie d'irradiation UV ou visible ou par voie de faisceau d'électrons, **caractérisée par le fait qu'**elle comprend au moins un produit réticulable (P) tel que défini à l'une des revendications 1 à 5.

**16.** Composition réticulable selon la revendication 15, **caractérisé par le fait qu'**elle comprend :

(a) au moins un produit réticulable (P) tel que défini à l'une des revendications 1 à 5 ;
(b) au moins un agent réticulant consistant en au moins un polythiol (I) tel que défini à l'une des revendications 1, 3, 4 et 5 ;
(c) en cas de réticulation par voie photochimique, le cas échéant un photoinitiateur ou un système photoinitiateur, auquel peut être associé un agent photoactivateur ;
(d) en cas de réticulation par voie thermique, un amorceur producteur de radicaux libres, auquel peut être associé un accélérateur ;
(e) le cas échéant, au moins un diluant réactif monomère ou oligomère ;
(f) le cas échéant, au moins un solvant ou diluant non réactif ; et
(g) le cas échéant au moins un additif usuel, tel qu'un pigment.

**17.** Films revêtant un substrat ou pièces moulées obtenus par la réticulation par voie radicalaire de la composition telle que définie à l'une des revendications 15 et 16.

**18.** Procédé de fabrication des produits tels que définis à la revendication 17 sous forme de films, **caractérisé par le fait qu'**on dépose la composition telle que définie à l'une des revendications 15 et 16 en couche mince sur un substrat et qu'on conduit la réticulation de cette composition par voie radicalaire.

**19.** Procédé de fabrication des produits tels que définis à la revendication 17 sous forme de pièces moulées, **caractérisé par le fait qu'**on introduit la composition dans un moule et qu'on conduit la réticulation de cette composition par voie radicalaire, le moule étant un moule transparent à au moins une partie des rayonnements de longueurs d'onde comprises entre 180 et 400 nm dans le cas où l'on utilise la voie photochimique.

**Patentansprüche**

**1.** Schwefelhaltiges Produkt, erhältlich durch Additionsreaktion von mindestens einem Polythiol (I) mit mindestens einer Verbindung der Formel (II):

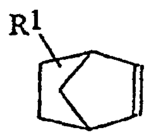

(II)

worin $R^1$ für einen linearen oder verzweigten aliphatischen Rest mit einer ethylenisch ungesättigten Doppelbindung steht, wobei $R^1$ über letztere an den Norbornenring gebunden sein kann und der Ring auch andere, unter $C_1$-$C_3$-Alkyl ausgewählte Substituenten enthalten kann, in Anteilen, die einer Thiolgruppe des bzw. der Polythiole (I) pro Mol der Verbindung bzw. Verbindungen (II) entsprechen, wobei die Thiolgruppen der Verbindung bzw. Verbindungen (I) sich hauptsächlich an die cyclische Doppelbindung der Verbindung bzw. Verbindungen (II) addieren und durchschnittlich eine der beiden ethylenischen Ungesättigtheiten einer Verbindung (II) frei bleibt.

**2.** Schwefelhaltiges Produkt nach Anspruch 1, **dadurch gekennzeichnet, daß** es sich bei der Verbindung der Formel (II) um 5-Vinyl-2-norbornen (5-Vinylbicyclo[2.2.1]hept-2-en) oder 5-Ethyliden-2-norbornen (5-Ethylidenbicyclo [2.2.1]hept-2-en) handelt.

**3.** Schwefelhaltiges Produkt nach Anspruch 1 oder 2, **dadurch gekennzeichnet, daß** die Polythiole (I) unter den Verbindungen der Formel $R^2$-[-SH]$_n$ ausgewählt sind, wobei:

- $R^2$ für einen aliphatischen, aromatischen oder heterocyclischen oder eine Kombination daraus enthaltenden n-wertigen Rest steht, der Substituenten enthalten kann, wobei das Gerüst oder die Substituenten durch mindestens ein, unter -O- oder -S- ausgewähltes Heteroatom und/oder eine unter

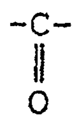, 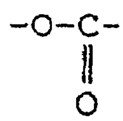 und 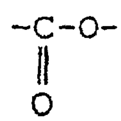

ausgewählte Gruppe unterbrochen sein können, und
- n für eine ganze Zahl von 2 bis 6 steht.

**4.** Schwefelhaltiges Produkt nach Anspruch 1, **dadurch gekennzeichnet, daß** es sich bei den Polythiolen (I) um Polythiole handelt, die durch Addition eines Polythiols der Formel $R^2$-[-SH]$_n$ nach Anspruch 3 an eine Verbindung der Formel (II) nach Anspruch 1 mit einem Thiolüberschuß und unter Additionsbedingungen, bei denen keine Vernetzung auftritt, erhältlich sind.

**5.** Schwefelhaltiges Produkt nach Anspruch 1, **dadurch gekennzeichnet, daß** das Polythiol (I) unter Dimercaptoethylsulfid, 4-Mercaptomethyl-3,6-dithia-1,8-ocandithiol, Pentaerythrittetrakis(3-mercaptopropionat) und Pentaerythrittetrakis(2-mercaptoacetat) ausgewählt ist.

**6.** Verfahren zur Herstellung eines schwefelhaltigen Produkts nach einem der Ansprüche 1 bis 5, **dadurch gekennzeichnet, daß** man bei einer Temperatur von 30 bis 170°C eine Mischung aus mindestens einem Polythiol (I) nach einem der Ansprüche 1 bis 5 und mindestens einer Verbindung (II) nach einem der Ansprüche 1 bis 5 zu einem vernetzbaren Produkt (P) umsetzt, wobei man die Anteile der Verbindungen (I) und (II) so wählt, daß eine Thiolgruppe der Verbindung bzw. Verbindungen (I) pro Mol der Verbindung bzw. Verbindungen (II) vorliegt, und eine Mischung aus dem so erhaltenen Produkt (P) und mindestens einer Verbindung der Formel (I) in stöchiometrischen Verhältnissen von Thiolgruppen zu Doppelbindungen auf radikalischem Wege zu einem Produkt (P') umsetzt, wobei die Radikale thermisch und/oder durch Bestrahlung mit UV oder sichtbarem Licht oder mit Hilfe eines Elektronenstrahls erzeugt werden können.

**7.** Verfahren nach Anspruch 6, **dadurch gekennzeichnet, daß** man die Umsetzung der Verbindungen (I) und (II) in Gegenwart von mindestens einem, unter radikalischen Initiatoren, wie Peroxiden, Peroxyestern, Peroxydicarbonaten und Azoverbindungen, sowie Säuren und Lewis-Säuren und Aminen ausgewählten Katalysator durchführt.

**8.** Verfahren nach Anspruch 6 oder 7, **dadurch gekennzeichnet, daß** man die Umsetzung der Verbindungen (I) und (II) ohne Lösungsmittel durchführt.

**9.** Verfahren nach einem der Ansprüche 6 bis 8, **dadurch gekennzeichnet, daß** man die Umsetzung der Verbindungen (I) und (II) in einem Lösungsmittelmedium durchführt, wobei das Lösungsmittel am Ende der Umsetzung entfernt wird.

**10.** Verwendung des Produkts (P) nach einem der Ansprüche 1 bis 5 in einer radikalisch vernetzbaren Zusammensetzung, die mindestens ein Polythiol(I) in stöchiometrischen Verhältnissen von Thiolgruppen des bzw. der Polythiole (I) zu Doppelbindungen des Produkts (P) enthält, wobei die Doppelbindungen des Produkts (P) vollständig verbraucht werden sollen, um ein vernetztes Produkt (P') zu erhalten.

**11.** Verwendung nach Anspruch 10, **dadurch gekennzeichnet, daß** man die Umsetzung des bzw. der Polythiole (I) mit dem Produkt (P) auf photochemischem Wege ohne Photoinitiator oder Photoinitiatorsystem oder in Gegenwart mindestens eines Photoinitiators oder Photoinitiatorsystems, der bzw. das in Kombination mit einem Photoaktivator vorliegen kann, durchführt.

**12.** Verwendung nach Anspruch 10 oder 11, **dadurch gekennzeichnet, daß** man die Umsetzung des bzw. der Polythiole (I) mit dem Produkt (P) auf thermischem Wege in Gegenwart mindestens eines radikalbildenden Initiators, der in Kombination mit einem Beschleuniger vorliegen kann, durchführt.

**13.** Verwendung nach einem der Ansprüche 10 bis 12, **dadurch gekennzeichnet, daß** man die Reaktionsmischung aus dem oder den Polythiolen (I) und dem Reaktionsprodukt (P) in dünner Schicht auf ein Substrat aufbringt und die Reaktion auf radikalischem Wege durchführt, wobei man ein vernetztes Produkt (P') in Form eines Films erhält.

**14.** Verwendung nach einem der Ansprüche 10 bis 12, **dadurch gekennzeichnet, daß** man die Reaktionsmischung aus dem oder den Polythiolen (I) und dem Reaktionsprodukt (P) in eine Form einbringt und die Reaktion auf radikalischem Wege durchführt, wobei man ein vernetztes Produkt (P') in Form eines Formteils erhält, wobei es sich bei Anwendung der photochemischen Route bei der Form um eine für mindestens einen Teil der Strahlung mit Wellenlängen zwischen 180 und 400 nm durchlässige Form handelt.

**15.** Radikalisch vernetzbare Zusammensetzung, wobei die Radikale thermisch und/oder durch Bestrahlung mit UV oder sichtbarem Licht oder mit Hilfe eines Elektronenstrahls erzeugt werden können, **dadurch gekennzeichnet, daß** sie mindestens ein vernetzbares Produkt (P) nach einem der Ansprüche 1 bis 5 enthält.

**16.** Vernetzbare Zusammensetzung nach Anspruch 15, **dadurch gekennzeichnet, daß** sie enthält:

(a) mindestens ein vernetzbares Produkt (P) nach einem der Ansprüche 1 bis 5;
(b) mindestens einen Vernetzer, der aus mindestens einem Polythiol (I) nach einem der Ansprüche 1, 3, 4 und 5 besteht;
(c) im Fall von photochemischer Vernetzung gegebenenfalls einen Photoinitiator oder ein Photoinitiatorsystem, der bzw. das in Kombination mit einem Photoaktivator vorliegen kann;
(d) im Fall von thermischer Vernetzung einen radikalbildenden Initiator, der in Kombination mit einem Beschleuniger vorliegen kann;
(e) gegebenenfalls mindestens ein monomeres oder oligomeres reaktives Verdünnungsmittel;
(f) gegebenenfalls mindestens ein Lösungsmittel oder nichtreaktives Verdünnungsmittel und
(g) gegebenenfalls mindestens ein übliches Additiv, wie ein Pigment.

**17.** Ein Substrat bedeckende Filme oder Formteile, die durch radikalische Vernetzung der Zusammensetzung nach Anspruch 15 oder 16 erhältlich sind.

**18.** Verfahren zur Herstellung der Produkte nach Anspruch 17 in Form von Filmen, **dadurch gekennzeichnet, daß** man die Zusammensetzung nach Anspruch 15 oder 16 in dünner Schicht auf ein Substrat aufbringt und sie radikalisch vernetzt.

**19.** Verfahren zur Herstellung der Produkte nach Anspruch 17 in Form von Formteilen, **dadurch gekennzeichnet, daß** man die Zusammensetzung in eine Form einbringt und sie radikalisch vernetzt, wobei es sich bei Anwendung der photochemischen Route bei der Form um eine für mindestens einen Teil der Strahlung mit Wellenlängen zwischen 180 und 400 nm durchlässige Form handelt.

## Claims

**1.** Sulphur product obtained by the addition reaction of at least one polythiol (I) with at least one compound of formula (II):

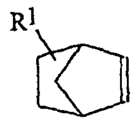 (II)

in which $R^1$ represents a linear or branched aliphatic residue comprising an ethylenic unsaturation double bond, it being possible for the latter to be that by which $R^1$ is connected to the norbornene ring, it also being possible for the said ring to comprise other substituents chosen from $C_1$-$C_3$ alkyl, in proportions corresponding to one thiol group of the polythiol or polythiols (I) per mole of the compound or compounds (II), the thiol groups of the compound or compounds (I) adding predominantly to the cyclic double bond of the compound or compounds (II) and, on average, one ethylenic unsaturation of a compound (II) of the two which it carries remaining free.

**2.** Sulphur product according to Claim 1, **characterized in that** the compound of formula (II) is 5-vinyl-2-norbornene (5-vinylbicyclo[2.2.1]hepto-2-ene) or 5-ethylidene-2-norbornene (5-ethylidene-bicyclo[2.2.1]hepto-2-ene).

**3.** Sulphur product according to either of Claims 1 and 2, **characterized in that** the polythiols (I) are chosen from the compounds of formula $R^2$-[-SH]$_n$ in which:

- $R^2$ is an n-valent aliphatic, aromatic or heterocyclic radical or an n-valent radical comprising a combination of these, it being possible for $R^2$ to comprise substituents, it being possible for the backbone or the substituents to be interrupted by at least one heteroatom chosen from -O- or -S- and/or one group chosen from

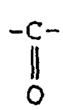 ; 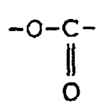 and 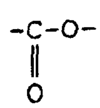 ;

and

- n is an integer from 2 to 6.

**4.** Sulphur product according to Claim 1, **characterized in that** the polythiols (I) are polythiols obtained by addition of a polythiol of formula $R^2$-[-SH]$_n$, as defined in Claim 3, to a compound of formula (II), as defined in Claim 1, with an excess of thiol and under addition conditions which prevent crosslinking.

**5.** Sulphur product according to Claim 1, **characterized in that** the polythiol (I) is chosen from dimercaptoethyl sulphide, 4-mercaptomethyl-3,6-dithia-1,8-octanedithiol, pentaerythritol tetrakis(3-mercaptopropionate) and pentaerythritol tetrakis(2-mercaptoacetate).

**6.** Process for the manufacture of the sulphur product as defined in one of Claims 1 to 5, **characterized in that** a mixture of at least one polythiol (I) as defined in one of Claims 1 to 5 and of at least one compound (II) as defined in one of Claims 1 to 5 is reacted at a temperature from 30 to 170°C, the proportions of the compounds (I) and (II) being chosen so that there is one thiol group of the compound or compounds (I) per one mole of the compound or compounds (II), in order to obtain the crosslinkable product (P), and that, in order to obtain the product (P'), a mixture of the product (P) thus obtained and of at least one compound of formula (I) is reacted by the radical route in stoichiometric proportions between the thiol groups and the double bonds, it being possible for the radicals to be generated thermally and/or by way of UV or visible irradiation or by way of an electron beam.

**7.** Process according to Claim 6, **characterized in that** the reaction of the compounds (I) and (II) is carried out in the presence of at least one catalyst chosen from free radical initiators, such as peroxides, peroxy esters, peroxydicarbonates and azo compounds, and acids and Lewis acids and amines.

**8.** Process according to either of Claims 6 and 7, **characterized in that** the reaction of the compounds (I) and (II) is carried out in the absence of solvent.

**9.** Process according to one of Claims 6 to 8, **characterized in that** the reaction of the compounds (I) and (II) is carried out in a solvent medium, the solvent being removed at the end of the reaction.

**10.** Use of the product (P) as defined in one of Claims 1 to 5 in a composition which can be crosslinked by the radical route comprising at least one polythiol (I) in stoichiometric proportions between the thiol groups of the polythiol or polythiols (I) and the double bonds of the said product (P), the double bonds of the said product (P) being intended to be completely consumed for the purpose of producing a crosslinked product (P').

**11.** Use according to Claim 10, **characterized in that** the reaction of the polyol or polyols (I) with the product (P) is carried out by the photochemical route in the absence of photoinitiator or photoinitiating system, or alternatively in the presence of at least one photoinitiator or of a photoinitiating system, which can be used in combination with a photoactivating agent.

**12.** Use according to either of Claims 10 and 11, **characterized in that** the reaction of the polythiol or polythiols (I) with the product (P) is carried out thermally, in the presence of at least one initiator, a producer of free radicals, which can be used in combination with an accelerator.

**13.** Use according to one of Claims 10 to 12, **characterized in that** the reaction mixture of the polythiol or polythiols (I) and of the reaction product (P) is deposited as a thin layer on a substrate and that the reaction is carried out by the radical route in order to obtain the crosslinked product (P') in the form of a film.

**14.** Use according to one of Claims 10 to 12, **characterized in that** the reaction mixture of the polythiol or polythiols (I) and of the reaction product (P) is introduced into a mould and that the reaction is carried out by the radical route in order to obtain the crosslinked product (P') in the form of a moulded item, the mould being a mould which is transparent to at least a portion of the radiation with wavelengths of between 180 and 400 nm in the case where the photochemical route is used.

**15.** Composition which can be crosslinked by the radical route, it being possible for the radicals to be generated thermally and/or by way of UV or visible irradiation or by way of an electron beam, **characterized in that** it comprises at least one crosslinkable product (P) as defined in one of Claims 1 to 5.

**16.** Crosslinkable composition according to Claim 15, **characterized in that** it comprises:

(a) at least one crosslinkable product (P) as defined in one of Claims 1 to 5;
(b) at least one crosslinking agent composed of at least one polythiol (I) as defined in one of Claims 1, 3, 4 and 5;
(c) in the event of crosslinking by the photochemical route, if appropriate a photoinitiator or a photoinitiating system, which can be used in combination with a photoactivating agent;
(d) in the event of crosslinking by the thermal route, an initiator which produces free radicals, which can be used in combination with an accelerator;
(e) if appropriate, at least one oligomer or monomer reactive diluent;
(f) if appropriate, at least one non-reactive diluent or solvent; and
(g) if appropriate, at least one conventional additive, such as a pigment.

**17.** Films coating a substrate or moulded items obtained by the crosslinking by the radical route of the composition as defined in either of Claims 15 and 16.

**18.** Process for the manufacture of the products as defined in Claim 17 in the form of films, **characterized in that** the composition as defined in either of Claims 15 and 16 is deposited as a thin layer on a substrate and that this composition is crosslinked by the radical route.

**19.** Process for the manufacture of the products as defined in Claim 17 in the form of moulded items, **characterized in that** the composition is introduced into a mould and that this composition is crosslinked by the radical route, the mould being a mould which is transparent to at least a portion of the radiation with wavelengths of between 180 and 400 nm in the case where the photochemical route is used.